# EUROPÄISCHE PATENTSCHRIFT

(11) **EP 3 681 605 B1**
(45) Veröffentlichungstag und Bekanntmachung des Hinweises auf die Patenterteilung: **07.06.2023**
(21) Anmeldenummer: 18773728.3
(22) Anmeldetag: 11.09.2018
(51) Int. Cl.: A62B 1/16, A62B 33/00, A62B 35/00, A41D 13/018, B63C 9/18, A43B 7/00, A41D 1/00, A41D 13/00, G08B 21/02, G08B 21/04, G08B 25/08

(54) **SCHUTZAUSRÜSTUNG MIT SENSOREINRICHTUNG**
PROTECTIVE EQUIPMENT COMPRISING A SENSOR DEVICE
ÉQUIPEMENT DE PROTECTION AVEC MOYEN DE DÉTECTION

(30) Priorität: 11.09.2017 DE 102017120925
(43) Veröffentlichungstag der Anmeldung: 22.07.2020
(73) Patentinhaber: Bornack GmbH & Co. KG, 74360 Ilsfeld (DE)
(72) Erfinder: BORNACK, Klaus, 74395 Mundelsheim (DE)
(74) Vertreter: Dilg, Haeusler, Schindelmann Patentanwaltsgesellschaft mbH
(86) Internationale Anmeldenummer: PCT/EP2018/074400
(87) Internationale Veröffentlichungsnummer: WO 2019/048692

(56) Entgegenhaltungen:
- EP-A1- 3 525 891
- US-A- 3 123 831
- US-A1- 2015 027 808
- US-A1- 2017 243 457

## Beschreibung

### Technisches Gebiet

Die vorliegende Erfindung betrifft eine Schutzausrüstung zum Schützen einer Person vor einer Notlage. Ferner betrifft die vorliegende Erfindung ein Verfahren zum Schützen einer Person vor einer Notlage.

### Hintergrund der Erfindung

Bei Arbeiten in gefährlichen Arbeitsumgebungen, wie beispielsweise in großer Höhe oder in kritischen Umgebungsbedingungen, besteht permanent die Gefahr, dass der Arbeiter in Notlagen gerät, welche das Leben des Arbeiters riskieren.

Beispielsweise kann bei Arbeiten unter geringen Umgebungstemperaturen der Arbeiter schnell eine Unterkühlung erlangen und benötigt eine Rettung aus einer entsprechenden Notlage. Ferner besteht bei Arbeiten in großen Höhen permanent das Risiko eines Absturzes. Um Personen diesbezüglich zu Sichern sind beispielsweise verschiedenste Arten von Absturzsicherungen gegeben. Beispielsweise werden verschiedene Seilsicherungssysteme eingesetzt, welche in einer Notlage einen Sturz der Person mechanisch abfangen sollen. Dies kann zu Verletzungen, wie beispielsweise Quetschungen oder Schleudertraumen, führen.

Zur Dämpfung des Aufprallstoßes sind beispielsweise Airbags in Motorrad-Schutzbekleidungen als Schutzpolsterung gegen ein Aufprallen auf der Straße bekannt. Ferner sind diesbezüglich Airbags in Autos als Schutzpolsterung zur Dämpfung eines Aufpralls bekannt.

US 3,123,831 A offenbart eine Schutzmaske, welche an einem Helm befestigbar ist. Eine Krempe ist an dem Helm befestigt. In der Krempe ist eine aufblasbare Tasche befestigt. Die Tasche kann aufgeblasen werden, beispielsweise mit einem Gas. Im aufgeblasenen Zustand schützt die Tasche das Gesicht des Benutzers. Zum Auslösen des Auflagevorgangs der Tasche kann ein photosensitiver Sensor dienen. Der photosensitive Sensor erfasst insbesondere auf nukleare Strahlung.

US 2017/243457 A1 offenbart eine Sturzsicherungsvorrichtung für Arbeiter auf einem Dach eines Gebäudes. Um die Ränder eines Daches wird ein Antennenkabel gespannt. Die Arbeiter tragen einen GPS Empfänger, welcher die Position des Arbeiters feststellt. Wenn der Arbeiter sich in eine Gefahrenzone nahe des Antennenkabels begibt, so wird ein Alarm, beispielsweise in seinem Helm, ausgelöst.

US 2015/027808 A1 offenbart eine Absturzsicherung für Hebebühnen. Ein Benutzer ist mit einem Sicherungsgeschirr an einer Sicherungsleine befestigt. Die Sicherungsleine ist an einem Ankerpunkt der Hebebühne befestigt. Die Sicherungsleine ist mit einem smarten Karabiner ausgestattet, welcher mit einer Überwachungseinheit oder zu einer anderen digitalen Vorrichtung kommuniziert. Der smarte Karabiner umfasst ferner einen Lastsensor, welcher feststellt, ob eine Belastungsgrenze, welche beispielsweise aufgrund des Herausfallens des Benutzers entsteht, überschritten wird. Zudem wird ein Lastsensor an die Sicherungsleine gekoppelt. Der Lastsensor übermittelt einen Alarm an die Sperrvorrichtung, wenn der Arbeiter von der Arbeitsplattform fällt und an dem Sicherungsgeschirr hängt.

### Darstellung der Erfindung

Es ist eine Aufgabe der vorliegenden Erfindung, bereits vor Eintreten einer Notlage eine Person zu schützen bzw. eine Notlage zu vermeiden.

Diese Aufgabe wird mit einer Schutzausrüstung zum Schützen einer Person in einer Notlage und einem Verfahren zum Schützen einer Person in einer Notlage gemäß den unabhängigen Ansprüchen gelöst.

Gemäß einem ersten Aspekt der vorliegenden Erfindung wird eine Schutzausrüstung zum Schützen einer Person vor einer Notlage beschrieben. Die Schutzausrüstung weist eine von der Person tragbare Sicherungsvorrichtung mit einem Sicherungssystem zum Schützen der Person vor einer Notlage auf, wobei die tragbare Sicherungsvorrichtung ein Anseilschutz, insbesondere ein Sicherungsgurt, ist. Ferner weist die Schutzausrüstung eine Sensoreinrichtung zum Feststellen einer Gefahrensituation vor der Notlage der Person auf, wobei die Sensoreinrichtung (direkt oder über eine Steuereinheit) mit dem Sicherungssystem derart gekoppelt ist, dass die Sensoreinrichtung selbst oder die Steuereinheit als Befehlsgeber für das Sicherungssystem fungieren, um bei Feststellen der Gefahrensituation der Person die Sensoreinrichtung das Sicherungssystem in einen Sicherungszustand zum Schützen der Person selbsttätig einstellt. Die Sensoreinrichtung weist einen Abstandssensor zum Messen eines Abstands zu einer Absturzkante als Gefahrenstelle auf. Das Sicherungssystem weist eine Fallsicherung auf, wobei die Fallsicherung eine Seilwinde aufweist, auf der ein Sicherungsseil aufgewickelt ist. Die Seilwinde der Fallsicherung ist mit dem Sicherungsseil ausgestattet und eingerichtet, die Länge einer Seillänge des Sicherungsseils zwischen der tragbaren Sicherungsvorrichtung und einem Sicherungspunkt zum Fixieren des Sicherungsseils zu steuern, wobei die Fallsicherung mit der Sensoreinrichtung derart gekoppelt ist, dass die Seillänge in Abhängigkeit von dem Feststellen der Gefahrensituation, bei welcher der Abstand der Person zu der Absturzkante unter einem Grenzwert ist, steuerbar ist.

Gemäß eines weiteren Aspekts der vorliegenden Erfindung wird ein Verfahren zum Schützen einer Person vor einer Notlage beschrieben. Das Verfahren weist auf ein Schützen einer Person mittels eines Sicherungssystems einer von der Person tragbaren Sicherungsvorrichtung, wobei die tragbare Sicherungsvorrichtung ein Anseilschutz, insbesondere ein Sicherungsgurt, ist. Ferner wird eine Gefahrensituation vor der Notlage der Person mittels einer Sensoreinrichtung festgestellt, welche mit dem Sicherungssystem gekoppelt ist. Die Sensoreinrichtung wird mit dem Sicherungssystem derart gekoppelt, dass die Sensoreinrichtung selbst oder die Steuereinheit als Befehlsgeber für das Sicherungssystem fungieren, um bei Feststellen der Gefahrensituation der Person die Sensoreinrichtung das Sicherungssystem in einen Sicherungszustand zum Schützen der Person selbsttätig einstellt. Die Sensoreinrichtung weist einen Abstandssensor zum Messen eines Abstands zu einer Absturzkante als Gefahrenstelle auf. Das Sicherungssystem weist eine Fallsicherung auf, wobei die Fallsicherung eine Seilwinde aufweist, auf der ein Sicherungsseil aufgewickelt ist. Die Seilwinde der Fallsicherung ist mit dem Sicherungsseil ausgestattet und eingerichtet, die Länge einer Seillänge des Sicherungsseils zwischen der tragbaren Sicherungsvorrichtung und einem Sicherungspunkt zum Fixieren des Sicherungsseils zu steuern, wobei die Fallsicherung mit der Sensoreinrichtung derart gekoppelt ist, dass die Seillänge in Abhängigkeit von dem Feststellen der Gefahrensituation, bei welcher der Abstand der Person zu der Absturzkante unter einem Grenzwert ist, steuerbar ist.

Eine Notlage definiert vorliegend eine Situation der Person, in welchen diese beispielsweise bereits verunfallt ist. Eine solche Notlage kann beispielsweise einen Absturz einer Person oder eine Unterkühlung einer Person darstellen. Die Gefahrensituation bezeichnet eine Situation insbesondere vor der Notlage. Mit anderen Worten wird mittels der Sensoreinrichtung bereits vor dem Eintreten einer Notlage die Gefahrensituation erkannt und der Sicherungszustand eingestellt. Die Gefahrensituation tritt beispielsweise ein, wenn ein Abstand der Person zu einer Absturzkante oder die Körpertemperatur der Person unter einem Grenzwert sind.

Der Sicherungszustand bezeichnet denjenigen Zustand, in welchen der Benutzer geschützt wird, sodass keine Notlage des Benutzers eintritt. In oben genannten Beispielen kann beispielsweise ein Sicherungsseil des Sicherungssystems gespannt werden, sodass ein Absturz vermieden wird, oder, wie unten beschrieben, kann eine Temperiereinrichtung die Person wärmen, um eine Unterkühlung (d. h. die Notlage) zu vermeiden.

Mit der vorliegenden Erfindung kann insbesondere die Sensoreinrichtung nach Feststellen der Gefahrensituation selbsttätig, d. h. ohne eine manuelle Intervention der Person oder einer weiteren Person, das Sicherungssystem aktivieren und in einen Sicherungszustand einstellen. Dazu kann die Sensoreinrichtung direkt mit dem Sicherungssystem oder indirekt über eine Steuereinheit gekoppelt werden. Die Sensoreinrichtung selbst oder die Steuereinheit fungieren als Befehlsgeber für das Sicherungssystem, um dieses in den Sicherungszustand einzustellen. Die Sensoreinrichtung sendet insbesondere elektrische, optische oder elektromagnetische Steuersignale an das Sicherungssystem. Die Sensoreinrichtung ist insbesondere eine elektronische Sensoreinrichtung.

Im Folgenden werden Beispiele für die Sensoreinrichtung, die Sicherungsvorrichtung und das Sicherungssystem erläutert: Zudem kann die Sensoreinrichtung zumindest einen Positionssensor, einen Beschleunigungssensor und/oder einen Bewegungssensor aufweisen.

Der Positionssensor ist beispielsweise ein GPS- Sensor, welcher GPS Daten ermittelt um eine exakte geographische bzw. räumliche Position der Person festzustellen. Befindet sich die Person beispielsweise in einer Gefahrensituation, beispielsweise in einem vorbestimmten Bereich vor einer Absturzkante, so kann daraus der Sicherungszustand des Sicherungssystems eingestellt werden.

Mittels des Beschleunigungssensors kann beispielsweise eine Position und/oder eine Beschleunigung der Person gemessen werden. Mit dem Bewegungssensor wird eine Geschwindigkeit der Person gemessen. Überschreitet die Person beispielsweise eine gewisse Geschwindigkeit, wie beispielsweise im Falle eines Absturzes, so tritt die definierte Gefahrensituation (schneller Fall) ein, sodass das Sicherungssystem den Sicherungszustand einnimmt, um einen weiteren Fall und somit einen Aufschlag (entspricht im Notfall) der Person am Boden verhindert.

Mittels des Positionssensors und/oder mittels des Beschleunigungssensors kann ferner die Lage (horizontal bzw. vertikal) der Person festgestellt werden. Liegt beispielsweise die Person in einer horizontalen Lage, so kann von einem gefahren Zustand der Person ausgegangen werden, sodass basierend darauf das Sicherungssystem aktiviert wird.

Ferner kann beispielsweise eine Wegänderung (horizontal oder vertikal) einer sich bewegenden Person, z.B. mittels des Positionssensors und/oder mittels des Beschleunigungssensors, gemessen werden. Bei Abweichung eines zulässigen Weges oder bei Verlassen eines Bewegungsfeldes der Person kann somit die Sensorvorrichtung das Sicherungssystem aktivieren.

Ferner kann die erfindungsgemäße Schutzausrüstung bzw. deren Sensoreinrichtung als sekundäres Sicherungssystem eingesetzt werden. So kann beispielsweise bei Versagen eines primären Sicherungssystems die erfindungsgemäße Schutzausrüstung als Notstopp fungieren. Fällt beispielsweise eine Fallsicherung als Primärsicherung, wie beispielsweise eine Anseilvorrichtung mit Fallstoppfunktion, welche bei einer bestimmten Fallgeschwindigkeit die Seillänge eines Sicherungsseils fixiert, aus, wird die sekundäre Sicherungsausrüstung gemäß der vorliegenden Erfindung aktiviert. Gleiches gilt beispielsweise für eine Abseilvorrichtung als Sicherungssystem. Wird die begrenzte Abseilgeschwindigkeit (welche mittels der Sensorvorrichtung gemessen wird) der Abseilvorrichtung überschritten, tritt ein Nothalt / zusätzliche Bremse in Aktion. Diesbezüglich kann beispielsweise die Sensoreinrichtung einen Abstand zum Boden detektieren und bei Unterschreiten eines Mindestabstandes das Sicherungssystem aktivieren. Das Sicherungssystem kann beispielsweise die oben beschriebene Fallsicherung der Primärsicherung sein, sodass die Sensoreinrichtung dann die Seillänge fixiert oder ein loses Sicherungsseil einholt und strafft.

Der Abstandssensor ist eingerichtet, einen Abstand zu einer vorgebbaren Referenzstelle und/oder einer Gefahrenstelle zu messen. Die Referenzstelle definiert beispielsweise eine Position im Raum, welche einen gewissen Abstand zu einer Gefahrenstelle, wie beispielsweise einem Hindernis oder einer Absturzkante, aufweist. Sobald der Abstand der Person zur Referenzstelle einen vorbestimmten Sollwert unterschreitet, wird beispielsweise selbsttätig das Sicherungssystem in den Sicherungszustand versetzt.

Die Sensoreinrichtung kann dabei beispielsweise einen optischen Sensor, einen Ultraschallsensor und/oder einen Infrarotsensor aufweisen, um z.B. einen Abstand zu einer vorgebbaren Referenzstelle und/oder einer Gefahrenstelle zu messen und/oder auch als Positionssensor zu fungieren.

Ferner kann die Sensoreinrichtung einen Höhensensor aufweisen, um eine Höhe über einer definierten Ebene oder Referenzfläche zu messen (beispielsweise über dem Boden bzw. über Meereshöhe). Die kritische Höhe kann beispielsweise einstellbar sein, so dass nach unterschreiten der kritischen Höhe beispielsweise selbsttätig das Sicherungssystem in den Sicherungszustand versetzt wird.

Gemäß einer weiteren beispielhaften Ausführungsform weist die Sensoreinrichtung einen medizinischen Sensor auf. Der medizinische Sensor ist eingerichtet, einen medizinischen Zustand, insbesondere die Körpertemperatur, die Atemfrequenz der Person und/oder die Herzfrequenz, zu messen. Der medizinische Sensor kann entsprechend beispielsweise ein Körperthermometer, ein Atemmessgerät, ein Pulsmessgerät und/oder ein Blutmessgerät zu messen von Blut werden, aufweisen. Arbeitet die Person beispielsweise in einer kühlen Arbeitsumgebung, so besteht beispielsweise die Gefahr der Unterkühlung (entspricht im Notfall). Unterschreitet die Körpertemperatur der Person eine gewisse Körpertemperatur, so tritt die Gefahrensituation ein. Die Sensoreinrichtung senden entsprechend Steuerbefehle an das Sicherungssystem, um einen Sicherungszustand einzustellen. Der Sicherungszustand kann beispielsweise eingestellt werden, in dem eine Körperheizung als Sicherungssystem aktiviert wird oder ein Alarm ausgesendet wird, um die Person aus der Gefahrensituation bzw. aus dem kalten Arbeitsumfeld herauszuholen, um die Person aufzuwärmen.

Somit kann eine Überanstrengung bzw. ein Kollaps der Person festgestellt werden und ein Sicherungszustand herbeigerufen werden.

Der medizinische Zustand kann an eine Aufsichtsperson übermittelt werden und/oder basierend auf dem medizinischen Zustand kann selbsttätig das Sicherungssystem in den Sicherungszustand versetzt werden.

Gemäß einer weiteren beispielhaften Ausführungsform weist die Sensoreinrichtung einen Umgebungssensor zum Messen von Umgebungsparametern, insbesondere der Umgebungstemperatur, des Umgebungswinds, des Umgebungsluftdruck und/oder der Umgebungsfeuchtigkeit, auf. Entsprechend kann die Sensoreinrichtung ein Thermometer, ein Windmessgerät, ein Barometer oder ein Hygrometer darstellen.

Ist die Person beispielsweise auf einer auf Off-Shore-Plattform oder einer Windturbinenanlage tätig, so kann die Person hohen Windgeschwindigkeiten und Stürmen ausgesetzt sein. Wird beispielsweise eine vordefinierte Grenzwindgeschwindigkeit mittels der Sensoreinrichtung gemessen, so kann beispielsweise ein Sicherungsseil als Sicherungssystem selbsttätig gestrafft werden, um die Sicherheit der Person zu gewährleisten.

Mittels der Messung des Luftdrucks kann beispielsweise eine Höhenveränderung (Sturzhöhe) festgestellt werden und ein Sicherungszustand eingestellt werden, indem beispielsweise ein Sicherungsseil gestrafft wird.

Das Sicherungssystem und/oder die Sensoreinrichtung sind beispielsweise in der Sicherungsvorrichtung integriert und befestigt, sodass die Person permanent mit der Sicherungsvorrichtung das Sicherungssystem und die Sensoreinrichtung mitträgt. Beispielsweise können das Sicherungssystem und die Sensoreinrichtung an einem Sicherungsgut als Sicherungsvorrichtung angeordnet sein. Die Person wird dabei in ihrer Bewegungsfreiheit nicht eingeschränkt.

Die Fallsicherung ist mit einem Sicherungsseil ausgestattet und eingerichtet, die Länge einer Seillänge des Sicherungsseils zwischen der tragbaren Sicherungsvorrichtung und einem Sicherungspunkt zum Fixieren des Sicherungsseils zu steuern. Die Fallsicherung ist mit der Sensoreinrichtung derart gekoppelt, dass die Seillänge in Abhängigkeit von dem Feststellen der Gefahrensituation steuerbar ist.

Dabei weist die Fallsicherung beispielsweise eine Seilwinde auf, welche beispielsweise elektrisch betreibbar ist. Auf der Seilwinde ist das Sicherungsseil aufgewickelt. Stellt die Sensoreinrichtung eine Gefahrensituation fest, beispielsweise falls ein Abstand zu einer Absturzstelle unterschritten wird oder eine Absturzgeschwindigkeit festgestellt wird, steuert die Sensoreinrichtung die Seilwinde in den Sicherungszustand. Dabei kann die Seilwinde beispielsweise die Länge des Sicherungsseils abrupt fixieren, indem die Seilwinde fixiert wird.

Zudem kann die Fallsicherung eine Seilbremse aufweisen, welche eine Abwickelgeschwindigkeit des Sicherungsseils von der Seilwinde reduziert.

Gemäß einer weiteren beispielhaften Ausführungsform weist das Sicherungssystem eine Airbageinrichtung auf. Die Airbageinrichtung ist aufblasbar ausgebildet, um im aufgeblasenen Zustand, d. h. in dem Sicherungszustand, einen Dämpfungskörper auszubilden. Die Airbageinrichtung ist mit der Sensoreinrichtung derart gekoppelt, dass die Airbageinrichtung in Abhängigkeit von dem Feststellen der Gefahrensituation aufblasbar ist. Wird beispielsweise ein Abstand zu einem Hindernis bzw. einer
Gefahrenstelle reduziert oder wird eine Absturzgeschwindigkeit festgestellt, aktiviert die Sensoreinrichtung die Airbageinrichtung. Da die Sensoreinrichtung bereits eine Gefahrensituation erkennt, welche zeitlich oder räumlich vor dem Eintreten der Notlage definiert ist, wird somit mehr Zeit zu Aktivierung der Airbageinrichtung ermöglicht, sodass ein besserer Schutz gegenüber einer reinen Aufprallerkennung, in welcher der Notfall bei Aktivierung des Airbags bereits eingetreten ist, erzielt wird.

Gemäß einer weiteren beispielhaften Ausführungsform ist die Airbageinrichtung als aufblasbare Schwimmweste ausgebildet. Beispielsweise kann die Sensoreinrichtung ein Feuchtigkeitsmessgerät aufweisen, welches die Umgebungsfeuchtigkeit in der Umgebung der Person misst. Stellt die Sensoreinrichtung beispielsweise fest, dass die Person in einem flüssigen Medium, wie beispielsweise Wasser, vorliegt, so kann die Airbageinrichtung als Schwimmweste aktiviert werden.

Gemäß einer weiteren beispielhaften Ausführungsform weist das Sicherungssystem eine Temperiereinrichtung auf, wobei die Temperiereinrichtung ausgebildet ist, um die tragbare Sicherungsvorrichtung zu temperieren. Die Temperiereinrichtung ist mit der Sensoreinrichtung derart gekoppelt ist, dass in Abhängigkeit von dem Feststellen der Notlage die Temperiereinrichtung steuerbar ist, um den Sicherungszustand zu erzielen. Die Temperiereinrichtung ist beispielsweise in der Sicherungsvorrichtung, beispielsweise in der Sicherungsjacke oder einem Sicherungsgut, derart integriert, dass die Temperiereinrichtung zwischen der Person und der Sicherungsvorrichtung vorliegt, um eine gute Wärmewirkung zu erzielen. Die Temperiereinrichtung kann beispielsweise eine Widerstandsheizung aufweisen.

Ferner kann die Temperiereinrichtung ein Temperiermedium aufweisen, welches durch entsprechende Temperierkanäle fließt, um eine Temperierwirkung zu erzielen. Dabei kann das Temperiermedium als Heizmedium oder Kühlmedium eingesetzt werden. Wird beispielsweise eine gewisse Unterkühlung der Person festgestellt, so kann die Temperiereinrichtung zur Beheizung der Person ausgebildet werden. Arbeitet die Person in einer heißen Umgebung, so kann beispielsweise bei Messung einer erhöhten Körpertemperatur die Temperiereinrichtung als Kühleinrichtung ausgelegt sein und entsprechend eine Kühlwirkung generieren. Die Temperiereinrichtung als Kühleinrichtung arbeitet beispielsweise nach dem Prinzip der Verdunstungskühlung und kann beispielsweise entsprechend einen Verdichter zum Verdichten des Kühlmediums aufweisen.

Gemäß einer weiteren beispielhaften Ausführungsform weist das Sicherungssystem eine Alarmeinrichtung auf. Die Alarmeinrichtung ist ausgebildet, ein Alarmsignal indikativ zur Gefahrensituation an eine Überwachungsstation zu senden. Die Alarmeinrichtung ist mit der Sensoreinrichtung derart gekoppelt ist, dass in Abhängigkeit von dem Feststellen der Notlage die Alarmeinrichtung das Alarmsignal indikativ zur Gefahrensituation sendet und der Sicherungszustand zum Schützen der Person einstellbar ist. Die Alarmeinrichtung kann beispielsweise ein optisches, akustisches oder elektrisches Warnsignal erzeugen. Die Überwachungsstation liegt räumlich entfernt von dem Sicherungssystem vor. Das Alarmsignal kann beispielsweise drahtlos an die Überwachungsstation gesendet werden.

Insbesondere kann die Sensoreinrichtung die Sensordaten an die Überwachungsstation senden. Darin kann beispielsweise eine Gefahrensituation oder eine Notlage durch eine aufsichtsführende Person festgestellt werden, auch wenn diese nicht vor Ort bei der Person ist. Somit wird mit anderen Worten eine Ferndiagnose möglich.

Gemäß einer weiteren beispielhaften Ausführungsform ist die Sensoreinrichtung mit dem Sicherungssystem derart gekoppelt, dass Sensordaten der Sensoreinrichtung drahtgebunden oder drahtlos übertragbar sind. Als drahtlose Übertragungstechnik ist beispielsweise eine NFC (Near Field Communication)- Verbindung, eine Funkverbindung mittels elektromagnetischer Wellen (Radiowellen) oder eine Transponderverbindung einsetzbar. Ferner können beispielsweise über optische Lichtwellenleiter, welche die Sensoreinrichtung mit dem Sicherungssystem koppeln, Signale übertragen werden.

Es wird darauf hingewiesen, dass die hier beschriebenen Ausführungsformen lediglich eine beschränkte Auswahl an möglichen Ausführungsvarianten der Erfindung darstellen. So ist es möglich, die Merkmale einzelner Ausführungsformen in geeigneter Weise miteinander zu kombinieren, so dass für den Fachmann mit den hier expliziten Ausführungsvarianten eine Vielzahl von verschiedenen Ausführungsformen als offensichtlich offenbart anzusehen sind. Insbesondere sind einige Ausführungsformen der Erfindung mit Vorrichtungsansprüchen und andere Ausführungsformen der Erfindung mit Verfahrensansprüchen beschrieben. Dem Fachmann wird jedoch bei der Lektüre dieser Anmeldung sofort klar werden, dass, sofern nicht explizit anders angegeben, zusätzlich zu einer Kombination von Merkmalen, die zu einem Typ von Erfindungsgegenstand gehören, auch eine beliebige Kombination von Merkmalen möglich ist, die zu unterschiedlichen Typen von Erfindungsgegenständen gehören.

### Kurze Beschreibung der Zeichnungen

Im Folgenden werden zur weiteren Erläuterung und zum besseren Verständnis der vorliegenden Erfindung Ausführungsbeispiele unter Bezugnahme auf die beigefügten Zeichnungen näher beschrieben. Es zeigen:
Fig. 1 eine schematische Darstellung einer Person mit einer Schutzausrüstung gemäß einer beispielhaften Ausführungsform der vorliegenden Erfindung.
Fig. 2 eine schematische Darstellung einer Schutzausrüstung mit einer Schutzjacke als Sicherungsvorrichtung gemäß einer beispielhaften Ausführungsform der vorliegenden Erfindung.

### Detaillierte Beschreibung von exemplarischen Ausführungsformen

Gleiche oder ähnliche Komponenten in unterschiedlichen Figuren sind mit gleichen Bezugsziffern versehen. Die Darstellungen in den Figuren sind schematisch.

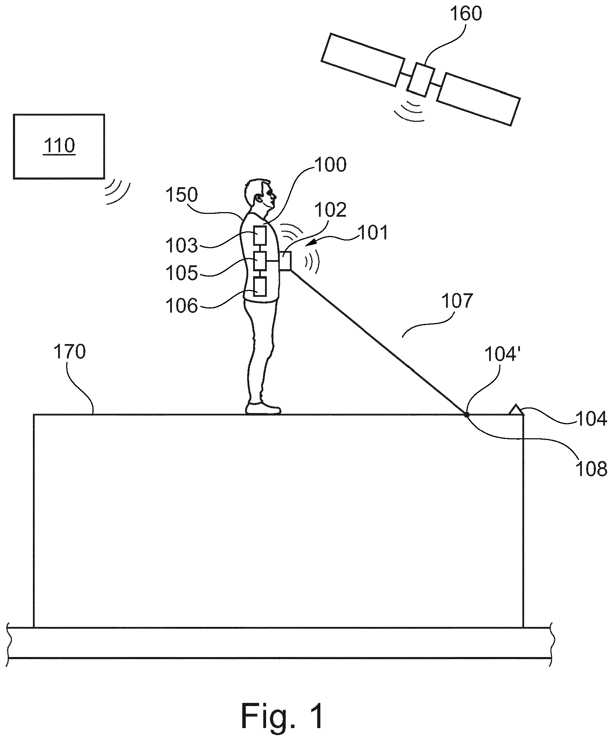

**Fig. 1** zeigt eine Schutzausrüstung 100 zum Schützen einer Person 150 vor einer Notlage. Die Schutzausrüstung 100 weist eine von der Person 150 tragbare Sicherungsvorrichtung 101 mit einem Sicherungssystem 102 zum Schützen der Person 150 vor einer Notlage auf. Ferner weist die Schutzausrüstung 100 eine Sensoreinrichtung 103 zum Feststellen einer Gefahrensituation vor der Notlage der Person 150 auf, wobei die Sensoreinrichtung 103 mit dem Sicherungssystem 102 derart gekoppelt ist, dass bei Feststellen der Gefahrensituation der Person 150 die Sensoreinrichtung 103 das Sicherungssystem 102 in einen Sicherungszustand zum Schützen der Person 150 selbsttätig einstellt.

In der beispielhaften Ausführungsform in Fig. 1 befindet sich die Person 150 auf einem Arbeitsbereich 170. Der Arbeitsbereich 170 stellt beispielsweise eine erhöhte Plattform, wie beispielsweise einen Container, dar. An den Kanten bzw. Rändern des Arbeitsbereichs 170 befindet wird eine Gefahrenstelle 104 definiert, da die Person 150 bei Überschreiten der Gefahrenstelle 104 über die Kante abstützen kann. Diesbezüglich kein ferner beabstandet von der Kante eine Gefahrenstelle 104'definiert werden. Somit kann bereits vor dem Absturz der Person 150 definiert werden, dass eine Gefahrensituation vorliegt und das Sicherungssystem aktiviert werden soll.

Die Person 150 in Fig. 1 trägt beispielsweise als Sicherungsvorrichtung 101 einen Anseilschutz, insbesondere einen Sicherungsgurt.

Das Sicherungssystem 102 weist eine Fallsicherung auf. Die Fallsicherung ist mit einem Sicherungsseil 107 ausgestattet und eingerichtet, die Länge einer Seillänge des Sicherungsseils 107 zwischen der tragbaren Sicherungsvorrichtung 101 und einem Sicherungspunkt 108 zum Fixieren des Sicherungsseils 107 zu steuern. Die Fallsicherung ist mit der Sensoreinrichtung 103 derart gekoppelt, dass die Seillänge in Abhängigkeit von dem Feststellen der Gefahrensituation steuerbar ist.

Dabei kann die Fallsicherung beispielsweise eine Seilwinde aufweisen, welche beispielsweise elektrisch betreibbar ist. Auf der Seilwinde ist das Sicherungsseil 107 aufgewickelt. Stellt die Sensoreinrichtung 103 eine Gefahrensituation fest, beispielsweise falls ein Abstand zu einer Absturzstelle (z.B. Abstand zwischen Referenzstelle 104' und Gefahrenstelle 104) unterschritten wird oder eine Absturzgeschwindigkeit festgestellt wird, steuert die Sensoreinrichtung 103 die Seilwinde in den Sicherungszustand. Dabei kann die Seitenwinde beispielsweise die Länge des Sicherungsseils 107 abrupt fixieren, indem die Seilwinde fixiert wird. Zudem kann die Fallsicherung eine Seilbremse aufweisen, welche eine Abwickelgeschwindigkeit des Sicherungsseils 107 von der Seilwinde reduziert.

Die Sensoreinrichtung weist zumindest einen Positionssensor, einen Beschleunigungssensor und/oder einen Bewegungssensor auf. Der Positionssensor ist beispielsweise ein GPS- Sensor, welcher GPS Daten von einem Satelliten 160 ermittelt, um eine exakte geographische bzw. räumliche Position der Person 150 festzustellen. Befindet sich die Person 150 beispielsweise in einer Gefahrensituation, beispielsweise an einer vorbestimmten Referenzstelle 104' vor einer Absturzkante 104, so kann daraus der Sicherungszustand des Sicherungssystems eingestellt werden.

Die Sensoreinrichtung 103 kann ferner einen Abstandssensor aufweisen. Der Abstandssensor ist eingerichtet, einen Abstand zu der vorgebbaren Referenzstelle 104' und/oder der Gefahrenstelle 104 zu messen. Die Referenzstelle 104' definiert beispielsweise eine Position im Raum, welche einen gewissen Abstand zu einer Gefahrenstelle 104, wie beispielsweise der Absturzkante, aufweist. Sobald der Abstand der Person 150 zur Referenzstelle einen vorbestimmten Sollwert unterschreitet, wird beispielsweise selbsttätig das Sicherungssystem 102, d.h. die Fallsicherung, in den Sicherungszustand versetzt.

Das Sicherungssystem 102 und/oder die Sensoreinrichtung 103 sind in der Sicherungsvorrichtung 101 integriert und befestigt, sodass die Person 150 permanent mit der Sicherungsvorrichtung 101, das Sicherungssystem 102 und die Sensoreinrichtung 103 mitträgt.

Insbesondere kann die Sensoreinrichtung 103 die Sensordaten an die Überwachungsstation 110 senden, beispielsweise mittels einer Übertragungseinheit 106. Darin kann beispielsweise eine Gefahrensituation oder eine Notlage durch eine aufsichtsführende Person festgestellt werden, auch wenn diese nicht vor Ort bei der Person 150 ist. Somit wird mit anderen Worten eine Ferndiagnose möglich.

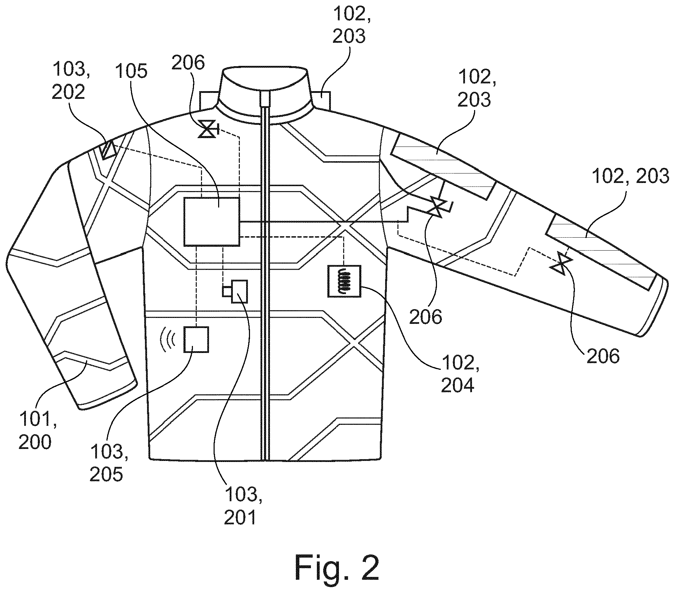

**Fig. 2** zeugt eine Schutzjacke 200 als Sicherungsvorrichtung 101. Das Sicherungssystem 102 und die Sensoreinrichtung 103 sind in der Schutzjacke 200 integriert.

Das Sicherungssystem 102 weist beispielsweise eine Airbageinrichtung 203 auf. Die Airbageinrichtung 203 ist aufblasbar ausgebildet, um im aufgeblasenen Zustand, d. h. in dem Sicherungszustand, einen Dämpfungskörper auszubilden. Die Airbageinrichtung 203 ist mit der Sensoreinrichtung 103 derart gekoppelt, dass die Airbageinrichtung 203 in Abhängigkeit von dem Feststellen der Gefahrensituation aufblasbar ist. Wird beispielsweise ein Abstand zu einem Hindernis bzw. einer Gefahrenstelle reduziert oder wird eine Absturzgeschwindigkeit festgestellt, aktiviert die Sensoreinrichtung 103 die Airbageinrichtung 203. Da die Sensoreinrichtung 103 bereits eine Gefahrensituation erkennt, welche zeitlich oder räumlich vor dem Eintreten der Notlage definiert ist, wird somit mehr Zeit zu Aktivierung der Airbageinrichtung 203 ermöglicht, sodass ein besserer Schutz gegenüber einer reinen Aufprallerkennung, in welcher der Notfall bei Aktivierung des Airbags bereits eingetreten ist, erzielt wird. Die Airbageinrichtung 203 kann beispielsweise mittels Steuern von Steuerelementen bzw. Ventilen 206 gesteuert werden.

Die Schutzjacke 200 weist beispielsweise einen medizinischen Sensor 201 als Sensoreinrichtung 103 auf. Der medizinische Sensor 201 ist eingerichtet, einen medizinischen Zustand, insbesondere die Körpertemperatur, die Atemfrequenz der Person 150 und/oder die Herzfrequenz, zu messen. Der medizinische Sensor 201 kann entsprechend beispielsweise ein Körperthermometer, ein Atemmessgerät, ein Pulsmessgerät und/oder ein Blutmessgerät zu messen von Blut werden, aufweisen. Arbeitet die Person 150 beispielsweise in einer kühlen Arbeitsumgebung, so besteht beispielsweise die Gefahr der Unterkühlung (entspricht im Notfall). Unterschreitet die Körpertemperatur der Person 150 eine gewisse Körpertemperatur, so tritt die Gefahrensituation ein. Der medizinische Sensor 201 sendet entsprechend Steuerbefehle an das Sicherungssystem 102, um den Sicherungszustand einzustellen.

Diesbezüglich weist das Sicherungssystem 102 beispielsweise eine Temperiereinrichtung 204 auf. Der Sicherungszustand kann beispielsweise eingestellt werden, in dem die Temperiereinrichtung 204 als Körperheizung ausgebildet wird, um die Körperwärme der Person 150 zu erhöhen.

Die Sensoreinrichtung 103 kann ferner eine in die Schutzjacke 200 integrierten Umgebungssensor 202 zum Messen von Umgebungsparametern, insbesondere der Umgebungstemperatur, des Umgebungswinds, des Umgebungsluftdruck und/oder der Umgebungsfeuchtigkeit, aufweisen. Mittels der Messung des Luftdrucks kann beispielsweise eine Höhenveränderung (Sturzhöhe) festgestellt werden und ein Sicherungszustand eingestellt werden, indem beispielsweise ein Sicherungsseil 107 (siehe Fig. 1) gestrafft wird.

Ferner ist in die Schutzjacke 200 eine Alarmeinrichtung 205 als Sicherungssystem 102 angeordnet. Die Alarmeinrichtung 205 ist ausgebildet, ein Alarmsignal indikativ zur Gefahrensituation an eine Überwachungsstation 110 (siehe Fig. 1) zu senden. Die Alarmeinrichtung 205 ist mit der Sensoreinrichtung 103 derart gekoppelt ist, dass in Abhängigkeit von dem Feststellen der Gefahrensituation die Alarmeinrichtung 205 das Alarmsignal generiert und z.B. der Sicherungszustand zum Schützen der Person 150 einstellbar ist. Die Alarmeinrichtung 205 kann beispielsweise ein optisches, akustisches oder elektrisches Warnsignal erzeugen. Die Überwachungsstation 110 ist räumlich entfernt von dem Sicherungssystem 102. Das Alarmsignal kann beispielsweise drahtlos an Überwachungsstation 110 gesendet werden.

Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden ist, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

### Bezugszeichenliste:

- 100: Schutzausrüstung
- 101: Sicherungsvorrichtung
- 102: Sicherungssystem
- 103: Sensoreinrichtung
- 104: Referenzstelle/Gefahrenstelle
- 105: Steuereinheit
- 106: Übertragungseinheit
- 107: Sicherungsseil
- 108: Sicherungspunkt
- 110: Überwachungsstation

- 150: Person
- 160: GPS-Satellit
- 170: Arbeitsbereich

- 200: Schutzjacke
- 201: medizinische Sensor
- 202: Umgebungssensor
- 203: Airbageinrichtung
- 204: Temperiereinrichtung
- 205: Alarmeinrichtung
- 206: Steuerelement/Ventil

## Patentansprüche

1. Schutzausrüstung (100) zum Schützen einer Person (150) vor einer Notlage, die Schutzausrüstung (100) aufweisend
eine von der Person (150) tragbare Sicherungsvorrichtung (101) mit einem Sicherungssystem (102) zum Schützen der Person (150) vor einer Notlage, wobei die tragbare Sicherungsvorrichtung (101) ein Anseilschutz, insbesondere ein Sicherungsgurt, ist,
eine Sensoreinrichtung (103) zum Feststellen einer Gefahrensituation vor einer Notlage der Person (150),
wobei die Sensoreinrichtung (103) direkt oder über eine Steuereinheit mit dem Sicherungssystem (102) derart gekoppelt ist, dass die Sensoreinrichtung (103) selbst oder die Steuereinheit als Befehlsgeber für das Sicherungssystem (102) fungieren, um bei Feststellen der Gefahrensituation der Person (150) die Sensoreinrichtung (103) das Sicherungssystem (102) in einen Sicherungszustand zum Schützen der Person (150) vor der Notlage selbsttätig einstellt,
wobei die Sensoreinrichtung (103) einen Abstandssensor zum Messen eines Abstands zu einer Absturzkante als Gefahrenstelle (104) aufweist,
wobei das Sicherungssystem (102) eine Fallsicherung aufweist,
wobei die Fallsicherung eine Seilwinde aufweist, auf der ein Sicherungsseil (107) aufgewickelt ist,
wobei die Seilwinde der Fallsicherung mit dem Sicherungsseil (107) ausgestattet ist und eingerichtet ist, die Länge einer Seillänge des Sicherungsseils (107) zwischen der tragbaren Sicherungsvorrichtung (101) und einem Sicherungspunkt (108) zum Fixieren des Sicherungsseils (107) zu steuern,
wobei die Fallsicherung mit der Sensoreinrichtung (103) derart gekoppelt ist, dass die Seillänge in Abhängigkeit von dem Feststellen der Gefahrensituation, bei welcher der Abstand der Person zu einer Absturzkante unter einem Grenzwert ist, steuerbar ist.

2. Schutzausrüstung (100) gemäß Anspruch 1,
wobei die Sensoreinrichtung (103) zumindest einen Positionssensoraufweist.

3. Schutzausrüstung (100) gemäß Anspruch 1 oder 2,
wobei die Sensoreinrichtung (103) einen Umgebungssensor (202) zum Messen von Umgebungsparametern, insbesondere der Umgebungstemperatur, des Umgebungswinds, des Umgebungsluftdruck und/oder der Umgebungsfeuchtigkeit, aufweist.

4. Schutzausrüstung (100) gemäß einem der Ansprüche 1 bis 3,
wobei das Sicherungssystem (102) eine Airbageinrichtung (203) aufweist,
wobei die Airbageinrichtung (203) aufblasbar ausgebildet ist, um im aufgeblasenen Zustand einen Dämpfungskörper auszubilden,
wobei die Airbageinrichtung (203) mit der Sensoreinrichtung (103) derart gekoppelt ist, dass die Airbageinrichtung (203) in Abhängigkeit von dem Feststellen der Gefahrensituation aufblasbar ist.

5. Schutzausrüstung (100) gemäß einem der Ansprüche 1 bis 4,
wobei das Sicherungssystem (102) eine Alarmeinrichtung (205) aufweist,
wobei die Alarmeinrichtung (205) ausgebildet ist, ein Alarmsignal indikativ zur Gefahrensituation an eine Überwachungsstation (110) zu senden,
wobei die Alarmeinrichtung (205) mit der Sensoreinrichtung (103) derart gekoppelt ist, dass in Abhängigkeit von dem Feststellen der Gefahrensituation die Alarmeinrichtung (205) das Alarmsignal indikativ zur Gefahrensituation sendet und der Sicherungszustand zum Schützen der Person (150) einstellbar ist.

6. Schutzausrüstung (100) gemäß einem der Ansprüche 1 bis 5,
wobei die Sensoreinrichtung (103) mit dem Sicherungssystem (102) derart gekoppelt ist, dass Sensordaten der Sensoreinrichtung (103) drahtgebunden oder drahtlos übertragbar sind.

7. Verfahren zum Schützen einer Person (150) vor einer Notlage, das Verfahren aufweisend
Schützen einer Person (150) mittels eines Sicherungssystems (102) einer von der Person (150) tragbaren Sicherungsvorrichtung (101), wobei die tragbare Sicherungsvorrichtung (101) ein Anseilschutz, insbesondere ein Sicherungsgurt, ist,
Feststellen einer Gefahrensituation vor der Notlage der Person (150) mittels einer Sensoreinrichtung (103), welche mit dem Sicherungssystem (102) gekoppelt ist, und
wobei die Sensoreinrichtung (103) direkt oder über eine Steuereinheit mit dem Sicherungssystem (102) derart gekoppelt wird, dass die Sensoreinrichtung (103) selbst oder die Steuereinheit als Befehlsgeber für das Sicherungssystem (102) fungieren, um bei Feststellen der Gefahrensituation der Person (150) die Sensoreinrichtung (103) das Sicherungssystem (102) in einen Sicherungszustand zum Schützen der Person (150) selbsttätig einstellt,
wobei die Sensoreinrichtung (103) einen Abstandssensor aufweist,
wobei der Abstandssensor , welcher einen Abstand zu einer Absturzkante als Gefahrenstelle(104) misst,
wobei das Sicherungssystem (102) eine Fallsicherung aufweist,
wobei die Fallsicherung eine Seilwinde aufweist, auf der ein Sicherungsseil (107) aufgewickelt ist,
wobei die Seilwinde der Fallsicherung mit dem Sicherungsseil (107) ausgestattet ist und eingerichtet ist, die Länge einer Seillänge des Sicherungsseils (107) zwischen der tragbaren Sicherungsvorrichtung (101) und einem Sicherungspunkt (108) zum Fixieren des Sicherungsseils (107) zu steuern,
wobei die Fallsicherung mit der Sensoreinrichtung (103) derart gekoppelt ist, dass die Seillänge in Abhängigkeit von dem Feststellen der Gefahrensituation, bei welcher ein Abstand der Person zu der Absturzkante unter einem Grenzwert ist, steuerbar ist.

## Claims

1. Protection equipment (100) for protecting a person (150) in an emergency, the protection equipment (100) comprising
a securing device (101) which is wearable by the person (150) with a securing system (102) for protecting the person (150) in an emergency, wherein the wearable securing device (101) is a rope protection, in particular a safety belt,
a sensor unit (103) for detecting a dangerous situation prior to an emergency of the person (150),
wherein the sensor unit (103) is coupled with the securing system (102) directly or via a control unit, such that the sensor unit (103) itself or the control unit function as a command provider for the securing system (102), such that, when detecting the dangerous situation for the person (150), the sensor unit (103) automatically adjusts the securing system (102) to a securing state for protecting the person (150) prior to the emergency,
wherein the sensor unit (103) comprises a distance sensor for measuring a distance to a falling edge as a danger location (104),
wherein the securing system (102) comprises a fall protection,
wherein the fall protection comprises a rope winch, on which a safety rope (107) is wound up,
wherein the rope winch of the fall protection is provided with the safety rope (107), and is configured to control the length of a rope length of the safety rope (107) between the wearable securing device (101) and a securing point (108) for fixing the safety rope (107),
wherein the fall protection is coupled with the sensor unit (103), such that the rope length is controllable depending on the detection of the dangerous situation, in which the distance of the person to a falling edge is below a boundary value.

2. Protection equipment (100) according to claim 1,
wherein the sensor unit (103) comprises at least one position sensor.

3. Protection equipment (100) according to claim 1 or 2,
wherein the sensor unit (103) comprises an environmental sensor (202) for measuring environmental parameters, in particular the environmental temperature, the environmental wind, the environmental pressure and/or the environmental humidity.

4. Protection equipment (100) according to one of the claims 1 to 3,
wherein the securing system (102) comprises an air bag unit (203),
wherein the air bag unit (203) is configured in an inflatable manner, to form a damping body in the inflated state,
wherein the air bag unit (203) is coupled with the sensor unit (103), such that the air bag unit (203) is inflatable depending on the detection of the dangerous situation.

5. Protection equipment (100) according to one of the claims 1 to 4,
wherein the securing system (102) comprises an alarm unit (205),
wherein the alarm unit (205) is configured to transmit an alarm signal which is indicative for the dangerous situation to a monitoring station (110),
wherein the alarm unit (205) is coupled with the sensor unit (103), such that, depending on the detection of the dangerous situation, the alarm unit (205) transmits the alarm signal which is indicative for the dangerous situation, and the securing state for protecting the person (150) is adjustable.

6. Protection equipment (100) according to one of the claims 1 to 5,
wherein the sensor unit (103) is coupled with the securing system (102), such that sensor data of the sensor unit (103) are transmittable in a wired or in a wireless manner.

7. Method for protecting a person (150) in an emergency, the method comprising
protecting a person (150) by a securing system (102) of a securing device (101) which is wearable by the person (150), wherein the wearable securing device (101) is a rope protection, in particular a safety belt,
detecting a dangerous situation prior to the emergency of the person (150) by a sensor unit (103) which is coupled with the securing system (102), and
wherein the sensor unit (103) is coupled with the securing system (102) directly or via a control unit, such that the sensor unit (103) itself or the control unit function as a command provider for the securing system (102), such that, when detecting the dangerous situation of the person (150), the sensor unit (103) automatically adjusts the securing system (102) to a securing state for protecting the person (150),
wherein the sensor unit (103) comprises a distance sensor,
wherein the distance sensor measures a distance to a falling edge as a danger location (104),
wherein the securing system (102) comprises a fall protection,
wherein the fall protection comprises a rope winch, on which a safety rope (107) is wound up,
wherein the rope winch of the fall protection is provided with the safety rope (107), and is configured to control the length of a rope length of the safety rope (107) between the wearable securing device (101) and a securing point (108) for fixing the safety rope (107),
wherein the fall protection is coupled with the sensor unit (103), such that the rope length is controllable depending on the detection of the dangerous situation, in which a distance of the person to the falling edge is below a boundary value.

## Revendications

1. Équipement de protection (100) pour protéger une personne (150) d'une situation d'urgence, l'équipement de protection (100) comprenant
un dispositif de sécurisation (101) qui peut être porté par la personne (150) comprenant un système de sécurisation (102) pour protéger la personne (150) d'une situation d'urgence, dans lequel le dispositif de sécurisation qui peut être porté (101) est une protection anti-chute, en particulier une sangle de sécurisation,
un dispositif de détection (103) pour détecter une situation de danger avant une situation d'urgence de la personne (150),
dans lequel le dispositif de détection (103) est couplé au système de sécurisation (102) directement ou par l'intermédiaire d'une unité de commande de telle sorte que le dispositif de détection (103) lui-même ou l'unité de commande agissent en tant que donneur d'ordre pour le système de sécurisation (102) afin de régler automatiquement le dispositif de détection (103) du système de sécurisation (102) dans un état de sécurisation pour protéger la personne (150) de la situation d'urgence lorsque la situation de danger de la personne (150) est détectée,
dans lequel le dispositif de détection (103) présente un capteur de distance pour mesurer une distance par rapport à un bord donnant sur le vide en tant que zone de danger (104),
dans lequel le système de sécurisation (102) présente une sécurité anti-chute,
dans lequel la sécurité anti-chute présente un treuil sur lequel un câble de sécurisation (107) est enroulé,
dans lequel le treuil de la sécurité anti-chute est équipé du câble de sécurisation (107) et est conçu pour commander la longueur d'une longueur de câble du câble de sécurisation (107) entre le dispositif de sécurisation pouvant être porté (101) et un point de sécurisation (108) pour une fixation du câble de sécurisation (107),
dans lequel la sécurité anti-chute est couplée au dispositif de détection (103) de telle sorte que la longueur de câble peut être commandée en fonction de la détermination de la situation de danger dans laquelle la distance de la personne par rapport à un bord donnant sur le vide est inférieure à une valeur limite.

2. Équipement de protection (100) selon la revendication 1,
dans lequel le dispositif de détection (103) présente au moins un capteur de position.

3. Équipement de protection (100) selon la revendication 1 ou 2, dans lequel le dispositif de détection (103) présente un capteur ambiant (202) pour mesurer des paramètres ambiants, en particulier la température ambiante, le vent ambiant, la pression atmosphérique ambiante et/ou l'humidité ambiante.

4. Équipement de protection (100) selon l'une quelconque des revendications 1 à 3,
dans lequel le système de sécurisation (102) comprend un dispositif de coussin gonflable (203),
dans lequel le dispositif de coussin gonflable (203) est conçu pour être gonflable afin de former un corps d'amortissement à l'état gonflé,
dans lequel le dispositif de coussin gonflable (203) est couplé au dispositif de détection (103) de telle sorte que le dispositif coussin gonflable (203) peut être gonflé en fonction de la détermination de la situation de danger.

5. Équipement de protection (100) selon l'une quelconque des revendications 1 à 4,
dans lequel le système de sécurisation (102) comprend un dispositif d'alarme (205),
dans lequel le dispositif d'alarme (205) est conçu pour envoyer un signal d'alarme indiquant une situation de danger à une station de surveillance (110),
dans lequel le dispositif d'alarme (205) est couplé au dispositif de détection (103) de telle sorte qu'en fonction de la détection de la situation de danger, le dispositif d'alarme (205) envoie le signal d'alarme indiquant la situation de danger et l'état de sécurisation peut être ajusté pour protéger la personne (150).

6. Équipement de protection (100) selon l'une quelconque des revendications 1 à 5,
dans lequel le dispositif de détection (103) est couplé au système de sécurisation (102) de telle manière que des données de détection du dispositif de détection (103) peuvent être transmises par fil ou sans fil.

7. Procédé de protection d'une personne (150) d'une situation d'urgence, le procédé présentant les étapes consistant à
protéger une personne (150) au moyen d'un système de sécurisation (102) d'un dispositif de sécurisation (101) porté par la personne (150), dans lequel le dispositif de sécurisation (101) qui peut être porté est une protection anti-chute, en particulier une sangle de sécurisation,
déterminer une situation de danger avant la situation d'urgence de la personne (150) au moyen d'un dispositif de détection (103) qui est couplé au système de sécurisation (102), et
dans lequel le dispositif de détection (103) est couplé au système de sécurisation (102) directement ou par l'intermédiaire d'une unité de commande de telle sorte que le dispositif de détection (103) lui-même ou l'unité de commande agissent en tant que donneur d'ordre pour le système de sécurisation (102) afin de régler automatiquement le dispositif de détection (103) du système de sécurisation (102) dans un état de sécurisation pour protéger la personne (150) lors de la détermination de la situation de danger de la personne (150),
dans lequel le dispositif de détection (103) présente un capteur de distance,
dans lequel le capteur de distance, qui mesure une distance par rapport à un bord donnant sur le vide en tant que zone de danger (104),
dans lequel le système de sécurisation (102) présente une sécurité anti-chute,
dans lequel la sécurité anti-chute présente un treuil sur lequel un câble de sécurisation (107) est enroulé,
dans lequel le treuil de la sécurité anti-chute est équipé du câble de sécurisation (107) et est conçu pour commander la longueur d'une longueur de câble du câble de sécurisation (107) entre le dispositif de sécurisation pouvant être porté (101) et un point de sécurisation (108) pour une fixation du câble de sécurisation (107),
dans lequel la sécurité anti-chute est couplée au dispositif de détection (103) de telle sorte que la longueur du câble peut être commandée en fonction de la détermination de la situation dangereuse dans laquelle une distance de la personne par rapport à un bord donnant sur le vide est inférieure à une valeur limite.
